# EUROPEAN PATENT SPECIFICATION

(11) **EP 0 469 127 B1**
(45) Date of publication and mention of the grant of the patent: **10.05.1995**
(21) Application number: 91904832.2
(22) Date of filing: 19.02.1991
(51) Int. Cl.: A61M 16/00

(54) **MEDICAL VENTILATOR AND METHOD OF USING A VENTILATOR**
BEATMUNGSGERÄT UND VERWENDUNGSVERFAHREN
RESPIRATEUR A USAGE MEDICAL ET METHODE DE L'USAGE

(30) Priority: 19.02.1990 GB 9003708
(43) Date of publication of application: 05.02.1992
(73) Proprietor: WRIGHT, Basil Martin, Rickmansworth, Hertfordshire WD3 3HG (GB); BAKER, John Albert, Rickmansworth, Hertfordshire WD3 3SF (GB)
(72) Inventor: WRIGHT, Basil Martin, Rickmansworth, Hertfordshire WD3 3HG (GB); BAKER, John Albert, Rickmansworth, Hertfordshire WD3 3SF (GB)
(74) Representative: Parker, Geoffrey
(86) International application number: GB9100248
(87) International publication number: WO9112042

(56) References cited:
- EP-A- 0 127 905
- WO-A-82/03326
- US-A- 4 635 631
- US-A- 4 838 257

## Description

This invention concerns medical ventilators and more particularly such ventilators for neonates.

Neonates are normally ventilated from the stem of a so-called T-piece of tubing to the top of which an air-oxygen or other appropriate gas mixture is applied at a steady rate. The mixture is exhausted by way of an expiratory valve which maintains a constant pressure in the system, and ventilation as such is effected by intermittent closure of the valve at a predetermined frequency unrelated to the neonate's own respiratory efforts. These efforts produce small, approximately sinusoidal pressure waves within the ventilator system which have seemed to offer a basis for triggering the ventilator to synchronise it with the neonate's respiration. In the past, several attempts to achieve such synchronisation in a manner suited to routine clinical usage proved unsuccessful because of the small size of the pressure waves and the limitations of technology at that time. More recently, further attempts using modern transducers and electronics have proved practicable. However, a subsequent comparison of this airway pressure triggered (APT) approach with others, involving airflow triggering or high frequency ventilation, led to a conclusion that the former approach had severe limitations.

An object of the present invention is to reduce these limitations.

In this connection it is to be noted that in the approach to APT to date, triggering of ventilation was to coincide with the positive peak of the detected pressure wave which was assumed to mark the onset of inspiration. This particular basis for triggering is used in various prior proposals, such as in US-A-4635631 , WO-A-8203326 and US-A-4838257. However, further study of the situation, from which study the invention arises, has shown this to be inappropriate for neonates.

In this study a reciprocating pump driven by a rotating crank was used to simulate a neonate and it was observed that an associated trigger signal for APT ventilation was out of phase with the pump. More specifically a switch operable by the pump to mark the onset of inspiration indicated that triggering was in fact occurring halfway through expiration, 90° out of phase. In retrospect this phase shift relative to the previously assumed respiration cycle can be explained by the extreme leakiness of neonatal ventilators compared to those for use with older subjects. This leakiness is consistent with the airway pressure waves reflecting not volume, but changes in flowrate, and the latter will be 90° out of phase with respiration.

In any event, on the basis of this study, it is proposed according to the invention that neonatal ventilation of airway pressure triggered form be operated substantially in synchronism with the zero-crossing point in the negative-going portion of each airway pressure cycle to coincide with the onset of natural inspriation.

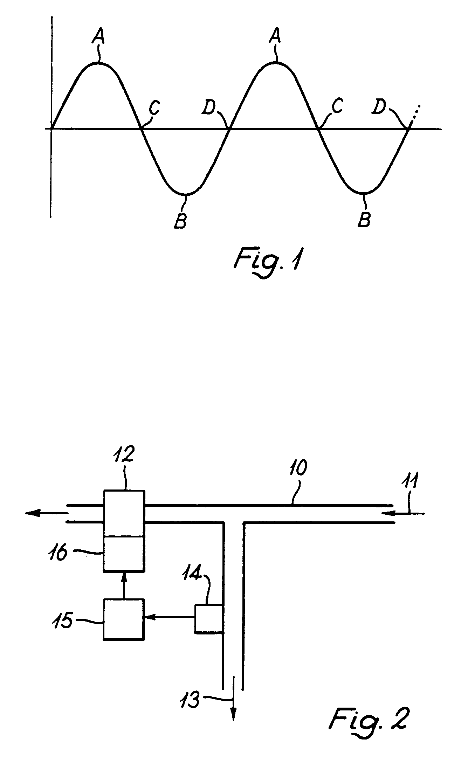

An understanding of the invention may be clarified by consideration of the accompanying drawings, in which:-
Figures 1 and 2 respectively schematically illustrate the detected airway pressure wave and the presently proposed ventilator.

The pressure wave of Figure 1 is shown as a sinusoidal variation with positive and negative peaks respectively denoted A and B. Prior attempts to effect APT neonatal ventilation have assumed this wave to represent respiration, with inspiration and expiration respectively occurring in the half cycles from A to B and B to A. Accordingly ventilation was triggered at A to coincide with the onset of inspiration.

However, as indicated above, respiration is now found to be 90° out of phase in a delayed sense with inspiration occurring in the half cycle from C to D. Ventilation should therefore be triggered at point C in the detected wave of Figure 1.

The ventilator of Figure 2 is largely as proposed before and includes a T-piece 10 across the top of which gas mixture 11 is applied at a steady rate to exhaust by way of an expiratory valve 12 operable to maintain a constant pressure in the piece 10. Ventilation to a neonate is effected by way of the stem, as Indicated at 13, intermittently in synchronism with the neonates' respiration efforts. These efforts are detected by a pressure transducer 14 which provides an output represented by Figure 1, this output is applied to a trigger circuit 15 to provide a trigger signal, and this last signal is applied in turn to a means 16 operable to close the valve 12 for a period appropriate to ventilation.

According to the invention the trigger circuit is augmented by some means whereby the trigger signal is provided substantially to coincide with point C on the output from transducer 14.

As so far developed, this means preferably involves a form of zero-crossing detection technique, the datum level for which represents the constant pressure which the expiratory valve acts to maintain in the system. However, other forms of means can be used.

## Claims

1. A method of using a ventilator of airway pressure triggered form characterised in that it is made operable for neonates substantially in synchronism with the zero-crossing point (C) in the negative-going portion (A-B) of each airway pressure cycle.

2. A method of using a ventilator comprising a generally T-shaped conduit (10) having a crossbar portion for receipt of pressurised respiration gas at one end thereof and a stem portion (13) extending transversely therefrom for application of said gas to a patient, an expiratory valve (12) at the other end of said crossbar portion which valve is operable to maintain a constant gas pressure in said conduit, a pressure transducer (14) connected with said stem portion to detect during use of said ventilator airway pressure variations therein due to natural respiration by said patient, and means (15,16) responsive to said pressure transducer to close said expiratory valve substantially in synchronism with a particular point in each cycle of said natural respiration, characterised in that said ventilator is made operable for said patient as a neonate with said point as the zero-crossing (C) in the negative-going portion (A-B) of said cycle.

3. A ventilator comprising a generally T-shaped conduit (10) having a crossbar portion for receipt of pressurised respiration gas at one end thereof and a stem portion (13) extending transversely therefrom for application of said gas to a patient, an expiratory valve (12) at the other end of said crossbar portion which valve is operable to maintain a constant gas pressure in said conduit, a pressure transducer (14) connected with said stem portion to detect during use of said ventilator airway pressure variations therein due to natural respiration by said patient, and means (15,16) responsive to said pressure transducer to close said expiratory valve substantially in synchronism with a particular point in each cycle of said natural respiration, characterised in that said stem portion is adapted for application to said patient as a neonate and said valve closing means includes a zero-crossing detector for which the datum level represents said constant gas pressure.

## Patentansprüche

1. Verfahren zur Verwendung eines durch Druck im Luftweg angesteuerten Beatmungsgeräts, dadurch gekennzeichnet, daß es für Neugeborene im wesentlichen synchron mit dem Nulldurchgangspunkt (C) des Negativanteils (A-B) eines jeden Luftweg-Druckzyklus betreibbar ist.

2. Verfahren zur Verwendung eines Beatmungsgeräts mit einer allgemein T-förmigen Leitung (10), die ein Querstück zur Aufnahme von Druckbeatmungsgas an seinem einem Ende und ein sich von diesem quer erstreckendes Stielteil (13) zur Abgabe des Gases an einen Patienten hat, einem zur Aufrechterhaltung eines konstanten Gasdrucks in der Leitung bedienbaren Ausatmungsventil (12) an dem anderen Ende des Querstücks, einem an den Stielteil angeschlossenen Druckwandler (14) zur Erfassung von während des Betriebs im Beatmungsgerät durch die natürliche Atmung des Patienten hervorgerufenen Druckänderungen im Luftweg, sowie mit einer auf den Druckwandler ansprechenden Vorrichtung (15, 16) zum Schließen des Ausatmungsventils im wesentlichen synchron mit einem bestimmten Punkt in jedem Zyklus der natürlichen Atmung, dadurch gekennzeichnet, daß das Beatmungsgerät bei einem Patienten betreibbar ist, der ein Neugeborenes ist, wobei der genannte Punkt dem Nulldurchgangspunkt (C) des Negativanteils (A-B) eines jeden Luftweg-Druckzyklus entspricht.

3. Beatmungsgerät mit einer allgemein T-förmigen Leitung (10), die ein Querstück zur Aufnahme von Druckbeatmungsgas an seinem einem Ende und ein sich von diesem quer erstreckendes Stielteil (13) zur Abgabe des Gases an einen Patienten hat, einem zur Aufrechterhaltung eines konstanten Gasdrucks in der Leitung bedienbaren Ausatmungsventil (12) an dem anderen Ende des Querstücks, einem an den stielteil angeschlossenen Druckwandler (14) zur Erfassung von während des Betriebs im Beatmungsgerät durch die natürliche Atmung des Patienten hervorgerufenen Druckänderungen im Luftweg, sowie mit einer auf den Druckwandler ansprechenden Vorrichtung (15, 16) zum schließen des Ausatmungsventils im wesentlichen synchron mit einem bestimmten Punkt in jedem Zyklus der natürlichen Atmung, dadurch gekennzeichnet, daß das stielteil bei einem Patienten anwendbar ist, der ein Neugeborenes ist, und daß die Ventilschließvorrichtung einen Nulldurchgangsdetektor beinhaltet, dessen Bezugspegel der konstante Druck darstellt.

## Revendications

1. Procédé d'utilisation d'un ventilateur du type déclenché par pression de passage d'air, caractérisé en ce qu'il est rendu actionnable pour des nouveaux-nés sensiblement en synchronisme avec le point de passage par zéro (C) de la partie à lancée négative (A-B) de chaque cycle de pression de passage d'air.

2. Procédé d'utilisation d'un ventilateur comportant un conduit (10) en forme générale de T possédant une branche transversale pour la réception de gaz de respiration pressurisé à l'une de ses extrémités et une jambe (13) orthogonale à ladite branche pour une application dudit gaz à un malade, une soupape d'expiration (12) à l'autre extrémité de ladite branche transversale, soupape qui est actionnable pour maintenir une pression de gaz constante dans ledit conduit, un transducteur de pression (14) relié à ladite jambe pour détecter, durant l'utilisation dudit ventilateur, des variations de pression du passage d'air à l'intérieur dûes à la respiration naturelle dudit malade, et des moyens (15, 16) sensibles audit transducteur de pression pour fermer ladite soupape d'expiration sensiblement en synchronisme avec un point particulier de chaque cycle de ladite respiration naturelle, caractérisé en ce que ledit ventilateur est rendu actionnable pour ledit malade nouveau-né avec ledit point en tant que passage par zéro (C) dans la partie à lancée négative (A-B) dudit cycle.

3. Ventilateur comportant un conduit (10) en forme générale de T possédant une branche transversale pour la réception du gaz de respiration pressurisé à l'une de ses extrémités et une jambe (13) s'étendant orthogonalement à celle-ci pour l'application dudit gaz à un malade, une soupape d'expiration (12) à l'autre extrémité de ladite branche transversale, ladite soupape étant actionnable pour maintenir une pression de gaz constante dans ledit conduit, un transducteur de pression (14) relié à ladite jambe pour détecter, durant une utilisation dudit ventilateur, des variations de pression de passage d'air à l'intérieur dûes à la respiration naturelle dudit malade, et des moyens (15, 16) sensibles audit transducteur de pression pour fermer ladite soupape d'expiration sensiblement en synchronisme avec un point particulier dans chaque cycle de ladite respiration naturelle, caractérisé en ce que ladite jambe est conçue pour une application audit malade en tant que nouveau-ne et lesdits moyens de fermeture de la soupape comprennent un détecteur de passage par zéro pour lequel le niveau de données représente ladite pression de gaz constante.
